# EUROPÄISCHE PATENTANMELDUNG

(11) **EP 4 371 545 A1**
(43) Veröffentlichungstag der Anmeldung: **22.05.2024**
(21) Anmeldenummer: 23209862.4
(22) Anmeldetag: 14.11.2023
(51) Int. Cl.: A61G 7/05, G16H 40/00

(54) **VORRICHTUNG ZUR BESTIMMUNG EINER POSITION EINES SEITENTEILS EINES BETTS**

(30) Priorität: 15.11.2022 DE 202022106414 U
(71) Anmelder: DewertOkin GmbH, 32278 Kirchlengern (DE)
(72) Erfinder: HILLE, Armin, 33659 Bielefeld (DE)
(74) Vertreter: Kleine, Hubertus

(57) **Zusammenfassung**

Die Erfindung betrifft eine Vorrichtung zur Bestimmung einer Position eines Seitenteils (4) eines Betts (1), insbesondere eines Pflege- oder Klinikbetts. Die Vorrichtung zeichnet sich dadurch aus, dass am Bett (1) im Bereich des Seitenteils (4) ein Sensor (11) angeordnet ist, der die Position des Seitenteils (4) berührungslos erfasst.

## Beschreibung

Die Erfindung betrifft eine Vorrichtung zum Erfassen einer Position eines Seitenteils eines Betts, insbesondere eines Pflege- oder Klinikbetts.

Um ein Herausfallen oder Heraussteigen einer Person, insbesondere einer kranken oder pflegebedürftigen Person, aus einem Bett zu verhindern, werden Betten mit anmontierbaren Seitenteilen, z.B. Seitengittern, eingesetzt. Zum Einsteigen in das Bett oder Aufstehen aus dem Bett oder wenn Seitenteile aus anderen Gründen nicht benötigt werden, können die Seitenteile in der Regel heruntergeklappt oder herabgesenkt werden.

Es ist bekannt, am Bett Schalter vorzusehen, die von den Seitenteilen betätigt werden und so eine Position der Seitenteile detektieren. Auf diese Weise kann ein Warnsignal generiert werden, wenn ein Seitenteil heruntergeklappt bzw. abgesenkt wird oder es z.B. um eine bestimmte Uhrzeit noch heruntergeklappt bzw. abgesenkt ist. Die Verwendung von Schaltern ist mit einem hohen Justieraufwand bei der Anbringung der Schalter verbunden, um sicherzustellen, dass die Schalter durch das Seitenteil in seinen verschiedenen Positionen korrekt betätigt werden. Zudem sind solche Schalter i.d.R. über Zuleitungskabel mit einer Auswerteeinheit verbunden, wobei die Zuleitungskabel bedingt durch die Positionierung der Schalter an der Seite eines Betts unterhalb der Seitenteile schwierig zu verlegen und zu reinigen sind und u.U. Knickstellen aufweisen, die zu einem Drahtbruch führen können.

Es ist eine Aufgabe der vorliegenden Erfindung, eine Vorrichtung der eingangs genannten Art zu schaffen, mit der eine Position eines Seitenteils eines Betts möglichst ohne großen Justieraufwand sicher erkannt wird und die auf einfach Weise am Bett montiert werden kann.

Diese Aufgabe wird gelöst durch eine Vorrichtung mit den Merkmalen des unabhängigen Anspruchs. Vorteilhafte Ausgestaltungen und Weiterbildungen sind Gegenstand der abhängigen Ansprüche.

Eine erfindungsgemäße Vorrichtung zur Bestimmung einer Position eines Seitenteils eines Betts zeichnet sich dadurch aus, dass am Bett im Bereich des Seitenteils ein Sensor angeordnet ist, der die Position des Seitenteils berührungslos erfasst.

Eine solche Sensoranordnung kann einfach z.B. am Kopfteil oder Fußteil des Betts positioniert werden und benötigt zur Montage lediglich zum Beispiel eine Federklemme, Schraubklemme, Klettband, doppelseitiges Klebeband oder ähnliches. Dadurch, dass die Position des Seitenteils berührungslos erfasst wird, ist z.B. gegenüber einem detektierenden Schalter eine große Positionierungstoleranz gegeben.

In einer vorteilhaften Ausgestaltung der Vorrichtung umfasst der Sensor einen Sender und einen Empfänger für ein Signal in Reflexionsgeometrie. Dabei können der Sender und der Empfänger optisch arbeiten, insbesondere mit Infrarotlicht, oder auch akustisch arbeiten, insbesondere mit Ultraschall. Durch die Reflexionsgeometrie können Sender und Empfänger auf derselben Seite des Seitengitters angeordnet sein, was Montage und Verdrahtung vereinfacht. Um eine hohe Signalgüte des Empfängers zu gewähren, kann am Seitenteil mindestens ein Reflektor für das Signal des Senders angeordnet sein.

In einer Weiterbildung kann in der genannten Reflexionsgeometrie eine Laufzeitmessung zwischen einem Senden und einem Empfangen des Signals zur Messung einer Entfernung zum Seitenteil vorgesehen sein. Dadurch kann ggf. zwischen verschiedenen eingestellten Zwischenpositionen des Seitengitters unterschieden werden, z.B. wenn es sich um ein verschiebbares Seitengitter handelt.

In einer weiteren vorteilhaften Ausgestaltung der Vorrichtung ist der Sensor in einer Sensoranordnung angeordnet, die eine Ansteuerschaltung für den Sensor, eine Auswerteschaltung für den Sensor und/oder eine Kommunikationseinrichtung aufweist. In dieser Ausgestaltung wird der Installationsaufwand für die Sensoranordnung minimiert. Insbesondere wenn eine drahtlos arbeitende Kommunikationseinrichtung und eine Stromversorgungseinheit, z.B. eine Batterie, mit integriert ist, wird außer der Befestigung der Sensoranordnung, z.B. am Kopfteil des Betts, kein weiterer Installationsaufwand notwendig.

Die Erfindung wird nachfolgend anhand eines Ausführungsbeispiels mithilfe von Figuren näher erläutert.

Die Figuren zeigen:
- Figur 1: ein Beispiel eines Betts mit einem hochgeschobenen Seitenteil und einer erfindungsgemäßen Vorrichtung; und
- Figur 2: das Betts gemäß Figur 1 mit abgesenktem Seitenteil.

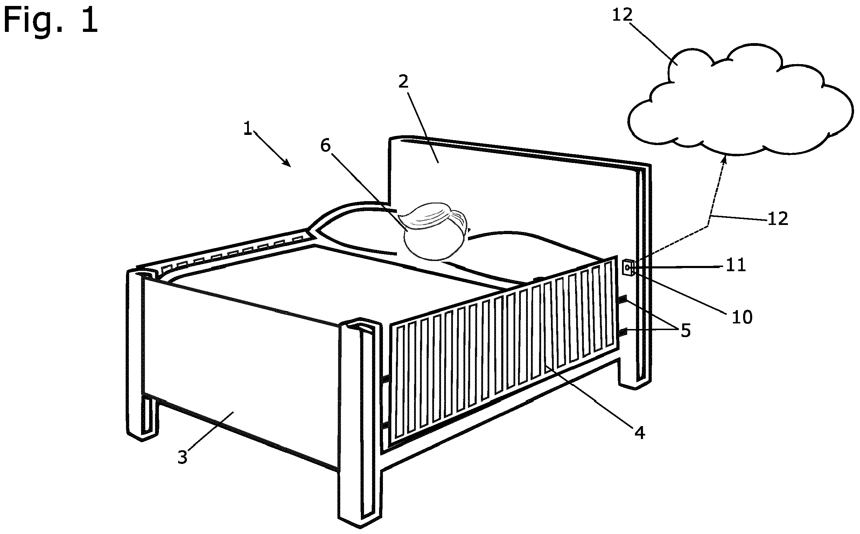

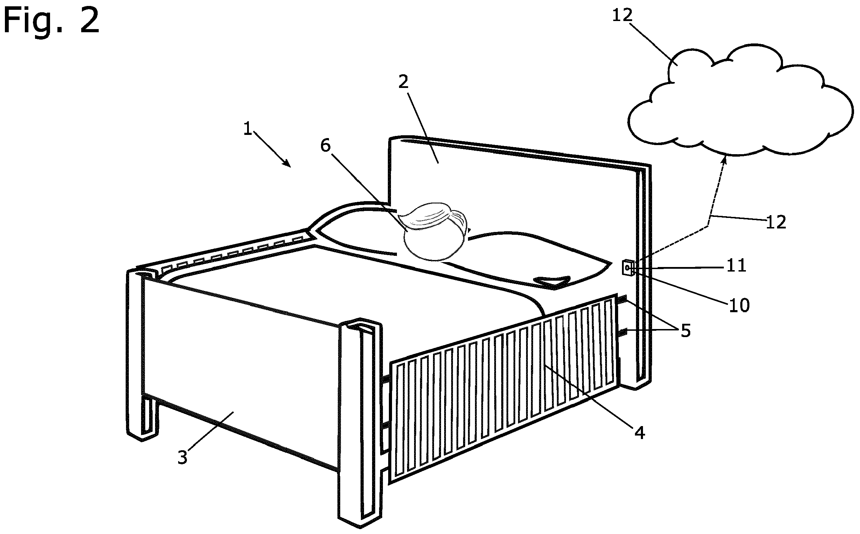

Die Figuren 1 und 2 zeigen in jeweils einer schematischen Darstellung ein Bett 1, insbesondere ein Pflege- oder Klinikbett, mit jeweils einem Kopfteil 2, einem Fußteil 3 und Seitenteilen 4. Die Seitenteile 4 sind über Führungen 5 in ihrer Höhe zumindest in eine hochgeschobene und eine abgesenkte Position einstellbar. Im Bett liegend ist schematisch eine Person 6 dargestellt.

Die Seitenteile 4 erstrecken sich bei dem dargestellten Beispiel über die jeweils gesamte Länge der Bettseite. Es wird angemerkt, dass die vorliegende Erfindung auch bei einem Bett umgesetzt sein kann, bei dem sich das Seitenteil nur über einen Teil der Länge des Betts erstreckt. Ein derartiges Seitenteil wird beispielsweise eingesetzt, wenn ein Herausfallen aus dem Bett verhindert werden soll, aber die Möglichkeit, das Bett zu verlassen, dadurch nicht eingeschränkt werden soll.

Die Figur 1 zeigt das Bett 1 mit dem Seitenteil 4 in einer hochgeschobenen Position und die Figur 2 das Bett 1 mit dem Seitenteil 4 in einer heruntergefahrenen Position.

Es ist eine Sensoranordnung 10 am Kopfteil 2 des Betts 1 montiert, die einen Sensor 11 aufweist, der so ausgerichtet ist, dass ein Raumbereich vor dem Sensor 11 auf Reflexionen durch das Seitenteil 4, insbesondere seine Stirnfläche überwacht wird. Es kann so die Position des Seitenteil 4 berührungslos erfasst werden. Dabei ist die Sensoranordnung 10 tolerant bezüglich ihrer Positionierung am Kopfteil 2 des Betts 1, wodurch eine aufwendige Justierung entfällt. Es ergibt sich vorteilhaft eine einfache Leitungsverlegung, ohne sich ständig bewegende Knick- und Drehpunkte.

Dazu weist der Sensor 11 einen Sender für ein Signal auf, dessen eventuelle Reflexion von einem ebenfalls im Sensor 11 enthaltenen Empfänger wieder registriert wird. Der Sensor 11 kann bevorzugt optisch arbeiten, insbesondere im Infrarot-Wellenlängenbereich, oder auch ein Ultraschallsensor sein. Es kann vorgesehen sein, dass die Reflexion am Seitenteil 4 selbst erfolgt, d.h. am (unveränderten) Material des Seitenteils. In der hochgeschobenen Position des Seitenteils 4 im Beispiel der Figur 1 erfolgt eine Reflexion am Seitenteil 4, bei der heruntergeschobenen Position gemäß Figur 2 nicht, was vom Sensor 11 auf diese Weise detektiert werden kann.

Es kann alternativ vorgesehen sein, an z.B. der dem Sensor 11 zugewandten Stirnseite des Seitenteils 4 einen Reflektor anzuordnen, der zu einem stärkeren Reflexionssignal am Sensor 11 führt, wenn sich das Seitenteil 4 in einer bestimmten Position befindet. Es kann dabei auch vorgesehen sein, mehrere Reflektoren zu nutzen, um auch Zwischenstellungen des Seitenteils 4 identifizieren zu können.

Alternativ zu der berührungslosen optischen oder akustischen Detektion der Position des Seitenteils 4 kann eine berührungslose Detektion auch magnetisch erfolgen, z.B. indem der Sensor 11 ein Reed-Schalter oder Hall-Sensor ist und am Seitenteil 4 Magnete angebracht sind.

Bei dem gezeigten Beispiel ist eine Sensoranordnung 10 nur auf einer Seite des Betts 1 genutzt, beispielsweise weil das Seitenteil auf der gegenüberliegenden Seite nicht absenkbar ist oder das Bett 1 an einer Wand steht. Es ist natürlich möglich, eine solche Sensoranordnung 10 an beiden Bettseiten vorzusehen.

In der Sensoranordnung 10 ist eine Steuer- und gegebenenfalls Auswerteschaltung für den Sensor 11 enthalten sowie eine Kommunikationseinrichtung. Die Kommunikationseinrichtung sendet die detektierte Positionsinformation des Seitenteils 4 über einen Kommunikationskanal 12 zum Beispiel in ein Netzwerk 13. Über das Netzwerk 13 kann eine Meldung der Sensoranordnung 10 an Warneinrichtungen weitergeleitet werden, die Pflege- oder Betreuungspersonal informieren. Die Warneinrichtungen können beispielsweise mobile Endgeräte auf Basis handelsüblicher Smartphones oder auch speziellen Krankenhaus-Pager sein. Alternativ oder zusätzlich kann ein akustisches oder optisches Warnsignal auch unmittelbar von der Sensoranordnung 10 ausgegeben werden.

Die Sensoranordnung 10 kann einfach am Kopfteil 2 positioniert werden und benötigt zur Installation lediglich zum Beispiel eine Federklemme, Schraubklemme, Klettband, doppelseitiges Klebeband oder ähnliches. Insbesondere bei einem drahtlosen Kommunikationskanal 12 ist nur ein geringer oder gar kein Verdrahtungsaufwand erforderlich.

In einer alternativen Ausgestaltung kann auch vorgesehen sein, die Sensoranordnung 10 an dem Fußteil 3 des Betts anzuordnen. Weiter ist auch eine Positionierung der Sensoranordnung 10 an einem Rahmen des Betts 1 denkbar, wobei der Sensor 11 nach oben ausgerichtet ist. In dem Fall kann vorgesehen sein, den Sensor 11 als Abstandssensor auszubilden, so dass eine (Schiebe-) Position des Seitengitters 4 berührungslos erfasst werden kann, wobei auch eingestellten Zwischenpositionen identifiziert werden können. Dieses kann beispielsweise über eine gemessene Signallaufzeit erfolgen. Der Sensor 11 kann in dem Fall bevorzugt ein Lidar (light detection and ranging)-Sensor sein oder auch ein Ultraschallsensor. Auch eine lichtbasierte Triangulationsmessung in Reflexionsgeometrie ist denkbar, um den Abstand zum Reflexionsort zu messen.

### Bezugszeichen

- 1: Bett
- 2: Kopfteil
- 3: Fußteil
- 4: Seitenteil
- 5: Führung
- 6: Person

- 10: Sensoranordnung
- 11: Sensor
- 12: Kommunikationskanal
- 13: Netzwerk

## Patentansprüche

1. Vorrichtung zur Bestimmung einer Position eines Seitenteils (4) eines Betts (1), insbesondere eines Pflege- oder Klinikbetts, **dadurch gekennzeichnet, dass**
am Bett (1) im Bereich des Seitenteils (4) ein Sensor (11) angeordnet ist, der die Position des Seitenteils (4) berührungslos erfasst.

2. Vorrichtung nach Anspruch 1, bei der der Sensor (11) einen Sender und einen Empfänger für ein Signal in Reflexionsgeometrie umfasst.

3. Vorrichtung nach Anspruch 2, bei der der Sender und der Empfänger optisch arbeiten, insbesondere mit Infrarotlicht.

4. Vorrichtung nach Anspruch 2, bei der der Sender und der Empfänger akustisch arbeiten, insbesondere mit Ultraschall.

5. Vorrichtung nach einem der Ansprüche 2 bis 4, bei der eine Laufzeitmessung zwischen einem Senden und einem Empfangen des Signals zur Messung einer Entfernung zum Seitenteil (4) vorgesehen ist.

6. Vorrichtung nach einem der Ansprüche 1 bis 5, bei der am Seitenteil (4) mindestens ein Reflektor für das Signal des Senders angeordnet ist.

7. Vorrichtung nach einem der Ansprüche 1 bis 6, bei der der Sensor (11) an einem Kopfteil (2) oder einem Fußteil (3) des Betts (1) montiert ist.

8. Vorrichtung nach einem der Ansprüche 1 bis 7, bei der der Sensor (11) in einer Sensoranordnung (10) angeordnet ist, die eine Ansteuerschaltung für den Sensor (11), eine Auswerteschaltung für den Sensor (11) und/oder eine Kommunikationseinrichtung aufweist.
